# EUROPEAN PATENT APPLICATION

(11) **EP 1 319 419 A1**
(43) Date of publication of application: **18.06.2003**
(21) Application number: 01403251.0
(22) Date of filing: 14.12.2001
(51) Int. Cl.: A61M 29/02, A61K 48/00

(54) **Method of administration of a gene of interest to a vascular tissue**

(71) Applicant: Aventis Pharma S.A., 92160 Antony (FR)
(72) Inventor: Duverger, Nicolas, 75005 Paris (FR); Branellec, Didier, 77170 Brie-Comte-Robert (FR); Aubailly, Nathalie, 33160 Saint Medard en Jalles (FR); Bilder, Glenda E., Lederach, PA 19450 (US); Bostwick, Jeffrey S., Upper Black Eddy, PA 18972 (US)
(74) Representative: Bouvet, Philippe

(57) **Abstract**

In a preferred embodiment such vector is an adenoviral vector.
This method can be used for the treatment of cardiovascular diseases, in particular hyperproliferative vascular disorders, such as restenosis, or ischemic diseases, such as peripheral artery diseases or coronary artery diseases, or atherosclerosis.

## Description

### Technical field

The present invention relates to a method of administration of a gene of interest to a vascular tissue comprising insertion into the vascular tissue an apparatus comprising a catheter and an inflatable balloon formed from a microporous membrane. It relates more particularly to the treatment of cardio-vascular diseases.

### Background

Percutaneous coronary interventions such as angioplasty, stent placement or atherectomy are effective therapies for the treatment of myocardial infarction and ischemic heart disease.
However, late reclosure or restenosis which occurs 6-12 months following these interventions remains a leading cause of revascularization failure, resulting in costly rehospitalization of 30-50% of patients for repeat procedures.
Over 50 clinical trials have failed to identify an effective therapy for restenosis and hence novel therapies are clearly needed
The exact mechanism(s) of restenosis remains poorly understood, but smooth muscle cell (SMC) migration/ proliferation/matrix production and vascular remodeling have been implicated in the process.
Gene therapy offers a hope of success.
Gene therapy, targeted against the cellular processes of SMC migration and/or proliferation, or arterial remodeling may provide an effective treatment for restenosis.
However, to be effective, this therapy must consider not only the appropriate gene but also a means of optimal local delivery.
Local delivery of genes or gene products at the site of angioplasty, stent placement or atherectomy may provide the means to precisely target the cells of interest and thereby achieve a higher concentration of the gene within the arterial wall than can be obtained by systemic administration. Furthermore, local catheter-based gene delivery would safeguard again systemic toxicity.
The catheter must deliver the gene locally with precision and without leakage to the systemic circulation. Additionally, the catheter must be operator friendly.

Previous results have been obtained with various apparatus but none of them showed any convincing evidence of their ability to deliver a gene of therapeutic interest to atherosclerotic arteries.
Specifically, the Infiltrator catheter® (Interventional Technologies) was used for adenoviral-mediated nitric oxide synthase (Varenne et al., Cir 1998:98:919-926, WO 98/34667) or C-type natriuretic (Morishige et al., J Am Coll Cardio 35:1040-1047, 2000) gene transfer to the injured coronary artery of the pig. However, the model system employed in these studies lacks relevance to man and neither Varenne et al.,(1998) nor Morishige et al., (2000) showed any evidence for the ability of the Infiltrator® catheter to deliver the adenoviral-gene complex to atherosclerotic arteries.

The Dispatch catheter® (Boston Scientific) was evaluated for adenoviral-mediated transfer of the marker gene, β-gal in the normal and atherosclerotic iliac artery of the rabbit (Tahlil et al., Cardiovas Res 1997:33:1810187). Although local delivery of the marker gene was achieved, there was evidence of viral leakage, raising the possibility of systemic toxicity with use of the Dispatch catheter. The performance of the Dispatch catheter® in a coronary artery was not evaluated.
Additionally, the hydrogel-coated balloon (Slider with Hydroplus, Mansfield Medical, Boston Scientific Corporation) was used to transfer adenoviral-β-gal to the normal rabbit iliac (Steg et al., Cir 1994:90:1648-1656) and the channel balloon (Palasis et al., Hum Gene Ther. 2000 ; 11:237-246 ; Boston Scientific Corporation) was used successfully to transfer adenoviral-Gax gene to the normal rabbit iliac simultaneous with balloon angioplasty (Maillard et al., Cardiovas Res 1997;35:536-546).
A recent report of local delivery devices compared the Infiltrator, the Crescendo, the Infusasleeve and the Channel balloon in the porcine coronary artery (Varenne O et al. Hum Gene Ther. 1999 ; 10:1105-1115). In this study, the authors showed that higher transduction levels were achieved in arteries with the Infiltrator, the Crescendo, and the Infusasleeve compared to the Channel balloon.
But still these apparatus have not shown any convincing evidence of their ability to deliver a gene of therapeutic interest to atherosclerotic arteries. Microporous balloons have been tested, according to application WO 98/34667, but the use of these balloons would results in "limited transgene expression".

Other apparatus comprising catheters have been described with regard to their use in delivery of drugs. But the possibility to use them to transfer genes of interest in arterial wall has never been disclosed.

### Summary

One object of the present invention is to provide a method for delivering a gene of interest to a vascular tissue comprising the following steps:
- inserting into said vascular tissue a catheter in fluid communication with an inflatable balloon formed from a microporous membrane, and
- delivering to the said vascular tissue through the catheter a solution containing a vector comprising a gene of interest and capable of expressing said gene into said vascular tissue.

In a preferred embodiment such vector is a viral vector, and more particularly an adenoviral vector.
Preferably the microporous membrane pores have an approximate size from about 10 Å to about 1µ and a pore density from about 10⁴ pores/cm² to about 10¹¹ pores/cm², and the balloon is inflated.
Preferably the vascular tissue is an artery.

In a particular aspect of said method according the vascular tissue is an atherosclerotic artery, or a vascular tissue connected with an ischemic tissue or an ischemic organ.

An other aspect of the present invention is a method for treating cardio-vascular disease comprising the following steps:
- inserting into a vascular tissue a catheter in fluid communication with an inflatable balloon formed from a microporous membrane, and
- delivering to the said vascular tissue through the catheter a solution containing a vector comprising a gene of interest and capable of expressing said gene into said vascular tissue.

Said method can be used for treating restenosis and comprises the following steps:
- inserting into an atherosclerotic artery a catheter in fluid communication with an inflatable balloon formed from a microporous membrane, and
- delivering to the said artery through the catheter a solution containing a vector comprising a suicide gene and capable of expressing said gene into said atherosclerotic artery.

Said suicide gene is a thymidine kinase gene or a cytosine desaminase gene.

Said method can also be used for treating an ischemic tissue or an ischemic organ and comprises the following steps:
- inserting into a vascular tissue connected with said ischemic tissue or ischemic organ a catheter in fluid communication with an inflatable balloon formed from a microporous membrane, and
- delivering to the said vascular tissue through the catheter a solution containing a vector comprising a gene encoding an angiogenic factor and capable of expressing said gene into said vascular tissue.

Said angiogenic factor is preferably FGF or VEGF.

Another object of the invention is the use of a vector comprising a gene of interest and capable of expressing said gene into said vascular tissue, for the manufacture of a medicament for administering a gene of interest to a vascular tissue by intravascular administration through an inflatable balloon formed from a microporous membrane.

A further object of the invention is the use of a vector comprising a gene of interest and capable of expressing said gene into said vascular tissue, for the manufacture of a medicament for use in the treatment of vascular diseases by intra vascular administration through an inflatable balloon formed from a microporous membrane.

Said disease can be restenosis or an ischemic disease.

Said gene can be a suicide gene, such as a thymidine kinase gene or a cytosine desaminase gene, or a gene encoding an angiogenic factor, such as FGFor VEGF.

Another object of the invention is an apparatus or a kit comprising:
- a catheter in fluid communication with an inflatable balloon formed from a microporous membrane, and
- a vector comprising a gene of interest.

Said apparatus can be used as medicament. It can be used as a combination for simultaneous or sequential use.

### Description of the drawings

Figure 1 : Adenovirus-mediated atheromatous rabbit iliac artery gene transfer with a Channel balloon delivery device (X30).
Figure 2 : Adenovirus-mediated atheromatous rabbit iliac artery gene transfer with a Channel balloon delivery device (X40).
Figure 3 : Adenovirus-mediated atheromatous rabbit iliac artery gene transfer with a e-Med microporous delivery device (X30).
Figure 4 : Adenovirus-mediated atheromatous rabbit iliac artery gene transfer with a e-Med microporous delivery device (X40).
Figure 5. Intensity of apparent β-Gal staining. N = 6 LAD & 6 LCX for the Annular and IVT groups; N = 8 LAD & 8 LCX for the e-Med and Channeled groups. Data are the mean ± sem.
Figure 6. Area of apparent β-Gal staining in the LAD & LCX coronary arteries. N = 6 LAD & 6 LCX for the Annular and IVT groups; N = 8 LAD & 8 LCX for the e-Med and Channeled groups. Data are the mean ± sem.
Figure 7A. Average total serum cholesterol at the time of balloon over-stretch and gene transfer. N = 30. * p < 0.05 (Student's t-test).
Figure 7B. Average total serum cholesterol level analyzed by treatment group. N = 6 for the Annular and IVT groups; N = 8 for the e-Med and Channeled groups. * P<0.05 vs. e-Med and Channeled; ** P<0.05 vs. Channeled (ANOVA). Data are the mean ± sem.
Figure 8. Delivery device balloon diameter to lumen diameter ratios. N = 6 LAD & 6 LCX for the Annular and IVT groups; N = 8 LAD & 8 LCX for the e-Med and Channeled groups. Data are the mean ± sem.
Figure 9. Number of branches per treated segment of artery. N = 27 (LAD), 26 (LCX). P<0.05 (Student's t-test). Data are the mean ± sem.
Figure 10 : Adenovirus-mediated porcine coronary artery gene transfer with a Channel balloon delivery device.
Figure 11 : Adenovirus-mediated porcine coronary artery gene transfer with a e-Med microporous balloon delivery device.

### Detailed description

### The microporous catheter

According to the present invention the vector comprising a gene of interest is administered to an arterial cell by intra arterial administration through an inflatable balloon formed from a microporous membrane.

In a preferred embodiment this microporous catheter is the one described in the US patent 5,569,198, and the PCT application WO 96/22805. The content of these patent and application is included by reference in the present application. The use of microporous catheters to deliver compounds to reduce vasomotor action, such as calcium antagonists, and inflammatory response is described, but not the use of these catheters to deliver vectors, and especially viruses.

According to a preferred embodiment said apparatus is the Microfuse Infusion Catheter (e Med Corporation, St Paul, Minnesota). This catheter is intended for localized delivery of solutions in the coronary arteries.
Such an apparatus is typically composed of two parts: the catheter part strictly speaking and the inflatable part formed from a porous membrane, the balloon. Both parts are in fluid communication through the lumen of the catheter.
The design of the microporous catheter according to the present invention permits appropriate contact of the balloon with the arterial wall such that within 45 seconds, vector-mediated gene transfer can occur.
In a preferred embodiment the pores size of the membrane is comprised between about 0.05 µ and about 1µ and the pore density is comprised between about 10⁶ pores/cm² and about 10⁹ pores/cm². More preferably the pores size of the membrane is about 1µ and the pore density is about 10⁶ pores/cm².
However the microporous catheter according to the present invention may be modified by an increase in the number of pores, a change in pore diameter, addition of a flow-through partition or strengthening the balloon to enable stent placement. These changes may serve to accommodate other future vectors, enhance the quantity of transferred gene and/or reduce procedure time by combining gene transfer with stent placement.

The microporous catheter is used as described in the US patent 5,569,198, and the PCT application WO 96/22805 or according to the manufacturer's instructions.

It can be used in the following manner. The guide wire is first inserted into the selected artery to a point past the desired position in the arterial system where the administration of vector should occur.
The catheter including the catheter body and the microporous balloon is then advanced along the guide wire to this desired position so that the microporous balloon is aligned with the target region of the artery.
The microporous balloon is then inflated by introducing an inflation fluid through the balloon lumen into the chamber. During inflation, the outer surfaces of the microporous balloon press outwardly against the inner surfaces of the vessel wall. Application of a pre determined pressure results in delivery of the solution containing the vector and transformation of the cells surrounding the vessel. The pressure inside the balloon is not great enough to cause more than a minimal amount of agent to escape from the balloon until the microporous balloon is in contact with the wall of the vessel.

Such a pressure is comprised between around 0.5 and around 20 atm (between around 7 and 294 p.s.i). It should be such as not to induce any alteration of the arterial wall. In a preferred embodiment such a pressure is comprised between around 1 and around 5 atm.

After delivery of the agent is complete, the microporous balloon is deflated and either removed from the patient's body or placed at a different location for treatment of another position.

### Vascular tissue

A vascular tissue means any tissue comprised within an internal tubular structure called a vessel that is connected to a tissue or organ within the body of a mammal, including man. Within the cavity of the tubular structure a bodily fluid flows to or from the body part. Examples of bodily fluid include blood or lymphatic fluid. Examples of vessels include arteries, arterioles, venules, sinusoids, veins and lymphatics. The intravascular route includes delivery through the blood vessels such as an artery or a vein.

Afferent blood vessels of organs are defined as vessels in which blood flows toward the organ or tissue under normal physiological conditions. Efferent blood vessels are defined as vessels in which blood flows away from the organ or tissue under normal physiological conditions. In the heart afferent vessels are known as coronary arteries, while efferent vessels are referred to as coronary veins.
In a preferred embodiment the vascular tissue is an artery and in particular a coronary artery.

### The vectors

The vectors according to the present invention can be viral vectors or non viral vectors, such as liposome based plasmid delivery systems or synthetic virus like systems.
In a preferred embodiment, the vectors according to the present invention are viral vectors. Among the later, there may be mentioned, *inter alia,* recombinant adenoviruses, recombinant adeno-associated viruses, recombinant retroviruses, such as MLV-derived or lentiviruses, herpesvirus, and vaccinia virus, hybrid viral vectors whose preparation may be carried out according to methods known to persons skilled in the art. Preferably, chimeric viral vectors are the adenovirus-retrovirus chimeric vectors which are described *inter alia* in application WO 95/22617, or the episome/adenovirus vectors which are described by Leblois et al. (*Mol Ther* (2000) **1(4)**, 314-322) and in Application WO 97/47757.
Preferred viral vectors are adenoviral vectors.

### The adenoviral vectors

Adenoviral vectors used according to the present invention are preferably vectors derived from defective adenoviruses, that is to say that they are incapable of autonomously replicating in the target cell. The construction of these defective viruses as well as their infectious properties have been widely described in the literature (see in particular S. Baeck and K.L. March, *Circul. Research,* **82,** (1998) 295-305); T. Shenk, B.N. Fields, D.M. Knipe, P.M. Howley et al. (1996), Adenoviridae: Viruses and Replication (in virology) 211-2148, EDS - Ravens publishers Philadelphia; Yeh, P. et al. *FASEB* **11** (1997) 615-623).

Various adenovirus serotypes, whose structure and properties vary somewhat have been characterized. Among these serotypes, use is preferably made in the context of the present invention of the type 2 or 5 human adenoviruses (Ad 2 or Ad 5) or adenoviruses of animal origin such as those described in Application FR 93 05954 or adenoviruses of mixed origin. Among the adenoviruses of animal origin which can be used in the context of the present invention, there may be mentioned the adenoviruses of canine, bovine, murine (Beard et al., *Virology* **75** (1990) 81), ovine, porcine, avian or simian origin. Preferably, the adenovirus of animal origin is a canine adenovirus, more preferably a CAV2 adenovirus (Manhattan or A26/61 strain) as described in Application WO 94/26914.

The defective adenoviruses of the invention comprise in general an inverted terminal repeat (ITR) at each end, a sequence allowing encapsidation (Psi), the E1 gene and at least one of the genes E2, E4 and L1-L5 having been moreover inactivated by any technique known to persons skilled in the art (Levero et al., *Gene,* **101** (1991) 195, EP 185 573; Graham, *EMBO J.* **3** (1984) 2917).

Advantageously, the recombinant adenovirus used in the context of the invention comprises a deletion in the E1 region of its genome. More particularly still, it comprises a deletion of the E1a and E1b regions. By way of precise example, there may be mentioned deletions affecting nucleotides 454-3328, 382-3446 or 357-4020 (with reference to the genome of Ad5).

According to a preferred variant, the recombinant adenovirus used in the context of the invention comprises, in addition, a deletion in the E4 region of its genome. More particularly, the deletion in the E4 region affects all the open reading frames. There may be mentioned, by way of precise example, the 33466-35535 or 33093-35535 deletions. Other types of deletions in the E4 region are described in applications WO 95/02697 and WO 96/22378, which are incorporated into the present by way of reference.

In another embodiment the adenovirus fiber protein or of the hexon has been modified to permit effective targeting of these adenoviral vectors. In particular the HRV5 loop of the hexon protein or the HI loop of the fiber protein (knob) can be modified as disclosed in the application WO 00/12738. Such vectors can be modified for targeting urokinase-type plasminogen activator receptor bearing cells.

In general, the recombinant adenoviruses according to the invention are formulated and administered in the form of doses of between 10⁴ and 10¹⁴ pfu, and preferably 10⁸ and 10¹² pfu. The term pfu (plaque forming unit) corresponds to the infectious power of a viral solution, and is determined by infecting an appropriate cell culture, and measuring the number of plaques of infected cells. The techniques for determining the pfu titre of a viral solution are well known in the art.

The viral solution may contain any pharmaceutically acceptable carrier or vehicle or excipient, such as saline buffer, isotonic solution, additives, stabilizers, etc.

### Other viral vectors

As regards the adeno-associated viruses (AAV), they are relatively small-sized DNA viruses which integrate into the genome of the cells which they infect, in a stable and site-specific manner. They are capable of infecting a broad spectrum of cells, without inducing any effect on cell growth, morphology or differentiation. Moreover, they do not appear to be involved in pathologies in humans. The AAV genome has been cloned, sequenced and characterized. It comprises about 4700 bases and contains, at each end, an inverted terminal repeat (ITR) of about 145 bases, serving as replication origin for the virus. The remainder of the genome is divided into 2 essential regions carrying the encapsidation functions: the left portion of the genome, which contains the rep gene involved in viral replication and the expression of the viral genes; the left portion of the genome, which contains the cap gene encoding the virus capsid proteins.

The use of AAV-derived vectors for the transfer of genes *in vitro* and *in vivo* has been described in the literature (see in particular WO 91/18088; WO 93/09239; US 4,797,368, US 5,139,941, EP 488528). These applications describe various AAV-derived constructs in which the rep and/or cap genes have been deleted and replaced with a gene of interest, and their use for transferring *in vitro* (on cells in culture) or *in vivo* (directly in an organism) the said gene of interest. The defective recombinant AAVs according to the invention may be prepared by co-transfection, into a cell line infected with a human helper virus (for example an adenovirus), of a plasmid containing the nucleic sequences of the invention bordered by two AAV inverted terminal repeats (ITR), and of a plasmid carrying the AAV encapsidation genes (rep and cap genes). The recombinant AAVs produced are then purified by conventional techniques.

Lentiviruses may also be used according to this embodiment; they allow the transfer and efficient and stable integration of a gene of interest into quiescent cells.

There may be mentioned for example HTLV-1 of animal lentiviruses such as FIV (feline infections virus), EIAV (equine infectious anemia virus; WO 98/51810), BIV (bovine immunodeficiency virus), SIV (simian immunodeficiency virus), CAEV (caprine arthritisencephalitis virus) (WO 98/39463; Naldini et al. *Science* **272** (1996) 263-267; Schnele et al. *Hum Gen Ther* **11** (2000) 439-447), or a lentivirus related to the one which causes AIDS, HIV-2 which is not highly pathogenic in humans (Kundra et al., *Hum Gen Ther* **9** (1998) 1371-1380).

### The gene of interest

The microporous catheter according to the present invention could be used for delivery of a variety of genes including those which inhibit proliferation, migration and/or matrix production of smooth muscle cells, those which inhibit focal shrinkage, those to inhibit macrophage infiltration and production of cytokines, those which retard the thrombogenicity of arterial wall, those to improve vasorelaxation, those which improve collateral arterial production, and those which prevent lipid uptake and/or oxidation.

Preferably, the gene of interest encodes a protein or an RNA which may be involved in cardiac pathologies such as cardiac insufficiency, cardiac hypertrophy, hypoxia, ischaemia or in cardiac transplant rejection.

As protein of therapeutic interest, there may be mentioned, *inter alia:*
- proteins inducing angiogenesis, such as for example members of the VEGF family, and more particularly VEGF-A, VEGF-B, VEGF-C or VEGF-D, members of the FGF family and more particularly FGF1, FGF2, FGF4, FGF5, angiogenin, EGF, TGFα, TGFβ, TNFα, Del-1, Scatter Factor/HGF, members of the angiopoietin family, cytokines and in particular interleukins including IL-1, IL-2, IL-8, angiotensin-2, plasminogen activator (TPA), urokinase (uPA), human tissue kallikrein, the molecules involved in the synthesis of active lipids (prostaglandins, Cox-1), factors inducing the production of angiogenic growth factors such as HIF and AKT/PKB;
- proteins involved in the control of cardiac contractility, such as phospholamban, phospholamban inhibitors, SERCA-2a, β2-adrenergic receptor or dystrophin or minidystrophin (FR 91 11947);
- proteins with cytoprotective activity, which in particular block apoptosis, such as proteins which are members of the bcl family, and protein kinases such as AKT/PKB;
- transcription factors, such as for example natural or chimeric nuclear receptors, comprising a DNA-binding domain, a ligand-binding domain and a transcription activating or inhibiting domain, such as for example the fusion proteins tetR-NLS-VP16, the fusion proteins derived from estrogen receptors, the fusion proteins derived from steroid hormone receptors, the fusion proteins derived from progesterone receptors, the proteins of the CID (Chemical Inducer of Dimerization) system described by Rivera et al., (Rivera et al., *Nature Medicine,* **2** (1996) 1028-1032). There may be mentioned in particular, as chimeric nuclear receptor, the nuclear receptors PPAR (Peroxisome Proliferator Activated Receptor) and in particular PPARγ2, as described in Applications WO 96/23884 and FR 99 07957 and by Frohnert et al., (*J Biol Chem* **274** (1999) 3970-3977), and Mukherjee et al., (*J Biol Chem* **272** (1997) 8071-8076) either in its native form, without modification of the primary structure, or a modified PPARγ2 comprising one or more ligand-binding sites or E/F domains (Schoonjans et al. *Biochim. Biophys. Acta*. **1302** (1996) 93-109), such as PPARγ2γ2 having the sequence SEQ ID NO: 3;
- immunosuppressors such as for example interleukins 2 and 10 which make it possible to completely or partially inhibit an immune signaling pathway and thus to extend the duration of cardiac transplants;
- proteins involved as agent for reducing hypoxia such as NOS (nitric oxide synthase), B-cell leukemia/lymphoma 2 (bcl-2), superoxide dismutase (SOD) and catalase.

As RNA of therapeutic interest, there may be mentioned for example antisense RNAs which are useful for controlling the expression of genes or the transcription of cellular mRNAs, thus blocking translation into a protein according to the technique described in Patent EP 140 308, as well as the ribozymes which are capable of selectively destroying target RNAs as described in EP 321 201.

It is understood that the present invention is not limited to the specific examples of proteins or RNAs, but that it can be used by persons skilled in the art for the expression of any nucleic acid in cardiac cells, by simple customary experimentation operations.

The gene of interest may be placed under the control of any suitable transcriptional promoter, preferably a promoter functional in mammalian cells, more preferably in human cells. The promoter may be constitutive, ubiquitous, regulated, inducible, tissue-specific, etc. The choice of a particular promoter can be made by the skilled person, depending on the disease to be treated.
Specific examples of promoters suitable for use in the present invention include promoters of viral origin (such as cytomegalovirus, respiratory syncitial virus, rous sarcoma virus, simian virus 40, retrovirus or lentivirus and the likes known in the states of art) of mammalian origin , house-keeping promoters (such as EF1alpha or E2F) or specifically expressed among which promoters of VEGF(s), FGF(s), HIF(s), Tie, VE cadherin, creatin kinase M, desmin, PGK, alpha smooth muscle cell actin, SM22, steel, kit, cardiac myosin, alpha myosin heavy chain, neuronal specific promoters and all promoter from all similar genes known by the skilled in the art person. Promoters could also be of synthetic origin such as inducible or regulated promoters (modulated by tetracycline, rapamycin, PPAR activator, hormone analogs, physiopathological situations...) or any promoters derived from fragments of the above mentioned using techniques known by the skilled person (deletion mutants, chimeric promoter, ...).

### Diseases to be treated

The method according to the present invention can be used for the treatment of any disease which is susceptible to be cured or prevented by the administration in a vascular tissue of a vector, and in particular an adenoviral vector, encoding a gene of therapeutic interest.
The tissue to be treated can be a vascular tissue but it can also be a non vascular tissue connected with this vascular tissue.
Such a non vascular tissue can be a muscle tissue, in particular a myocardial tissue or a skeletal muscle tissue.
The present method is thus intended for the treatment of cardio-vascular diseases.
In particular the present method is intended for the treatment of hyperproliferative vascular disorders, such as restenosis.
It is also particularly adapted for the treatment of ischemic diseases, such as peripheral artery disease or coronary artery disease. In a preferred embodiment the method according to the present invention comprises stimulating collateral vessel development in patients having myocardial ischemia.

Thus the method allows the treatment of diseases directly linked to vascular tissues, such as restenosis, but also of diseases linked to ischemic tissues such as myocardium, connected to a vessel, such as an artery.
For the treatment of diseases linked to an other organ or tissue than vascular tissues, the vector is administered into a vascular tissue in proximity of the organ or tissues to be treated. Thus the present method allows the treatment of any organ in the vicinity of vascular tissues or of any vascularized organ.

In a preferred embodiment the methods according to the present invention comprise a step of infusion of an adenoviral vector into a blood vessel that perfuses the heart.

The present invention is particularly intended to be used for the treatment of man.
However it can be adapted for the treatment of any animal, in particular of mammals.

### Treatment of restenosis

In a preferred embodiment the method according to the present invention is applied to the treatment of restenosis. In such a case an adenovirus comprising a suicide gene can be used.

According to the present invention a suicide gene is a gene, the expression of which provides to the infected cell a sensitivity to a therapeutic agent. The thymidine kinase gene, which renders the cells sensitive to some agents such as ganciclovir or acyclovir, or the cytosine desaminase gene, which renders the cells sensitive to 5-fluoro-cytosine (5-FC).

In a preferred embodiment the thymidine kinase gene of the human herpes virus (hTK HSV-1) is used. The sequence of this gene has already been disclosed (in particular by McKnight et al (Nucleic Acid. Res. 8 (1980) 5931).
It is also possible to use derivatives of this sequence having a more important substrate specificity or a best kinase activity.

Such derivatives may be obtained in particular by mutagenesis as described by Balasubramaniam et al., J. Gen. Virol. 71 (1990) 2979 and Munir et al., JBC 267 (1992) 6584.

It is also possible to use the cytosine désaminase gene, which confers to the cells a sensitivity to 5-fluoro-cytosine (5-FC).The cells which express this gene are able to convert 5-fluoro-cytosine (5-FC) in 5-fluoro-uracile (5-FU), which is a toxic métabolite. The sequence of this gene has been disclosed by Anderson et al. Arch. Microbiol. 152 (1989) 115.

An adenovirus comprising a suicide gene can be the adenovirus Ad-RSV-tk the construction of which is described in the PCT application WO 96/05321 (Rhône Poulenc Rorer).

To carry out the present invention, a person skilled in the art can advantageously refer to the following manual "SAMBROOK et al. (Molecular Cloning, A Laboratory Manual, Cold Spring Harbor Laboratory Press, New York 1989), or one of its recent editions.

The following examples illustrate particular embodiments of the present invention and are not limiting of the specification and claims in any way.

### Example 1 : Adenoviral-mediated β-Galactosidase gene transfer in the rabbit iliac artery model of stenosis

### 1. Summary

This report summarizes the methods and results from different studies which evaluated the ability of local delivery devices to deliver an adenoviral solution (AV 1.0 CMV β-Gal/ 10¹² viral particles per artery), in the rabbit iliac artery of normal and hypercholesterolemic animals. Devices used for local delivery were e-Med catheter, Channel Balloon and Annular balloon catheters. Gene transfer efficacy was determined by microscopic analysis of cells staining for β-Gal. Data indicate greater efficiency, higher cellular transduction and homogeneity over the artery segment, with the e-Med catheter in comparison to that obtained with the two other devices.

### 2. Purpose of the study

These studies evaluated the ability of e-Med Microporous, Channel balloon and Annular balloon local delivery devices to deliver a marker gene, β-Gal, encoded into an adenoviral vector (AV 1.0 CMV β-Gal / 10¹² virus particles (vp)/artery), to the arterial wall in atherosclerotic and normal rabbit iliac arteries.

### 3. Material and methods

### 3.1. Material and methods for gene transfer in normal rabbits

### 3.1.1 ANIMALS

- Species : Rabbits.
- Strain : New Zealand White.
- Sex: Male.
- Characterization :Conventional.
- Body weight range at initiation of treatment : 4,0 Kg.
- Experiments were reviewed and approved by internal ethical committee (Approval n°95-15).

### 3.1.2. TREATMENT

| **Adenovirus description** | | |
|---|---|---|
| Construct | Batch | Particle number |
| AV _{1.0} CMV β-Gal | V143 | 5.10¹² PV/ml |

- Buffer: PBS containing 10% glycerol
- Storage :- 80°C

### Adenovirus preparation

• Prepare PBS solution as followed:

| | |
|---|---|
| D-PBS pH7,4 | 45 ml |
| Glycerol 10% | 5 ml |
| MgCl₂ 0,5M 0,1% | 50 µl |

• Thaw rapidly the adenoviral aliquots at room temperature and dilute them in the PBS solution described above.
• Dilutions are prepared just before use in order to avoid any virus degradation with prolonged storage.

### Animal groups and treatment

- At day 0 :
   Rabbits are weighted.

◇ Anesthesia step :
   Following placement in the restraining cage and after disinfection, intramuscular injection of a mixture of Ketamine (50mg/kg) / Xylazine (5 mg/kg) is administered.
   A 22 gauge catheter is placed in the marginal ear vein and is flushed with sodium heparinate (120 Ul/kg).
◇ Injury step for Annular, Channel and e-Med Microfuse balloon gene transfer delivery:
   - Following placement in the restraining cage and after disinfection, intramuscular injection of a mixture of Ketamine (50mg/kg) / Xylazine (5 mg/kg) is administered. A 22 gauge catheter is placed in the marginal ear vein and is flushed with sodium heparinate (120 Ul/kg). Blood samples are taken.
   - A 5 Fr. introducer sheath (Vygon) is positioned in the carotid artery. All catheters are subsequently introduced through this sheath.
   - A 3 cm long 2.5 mm angioplasty balloon (Nycomed) is then advanced over a 0.014" guide wire (Boston Scientific). Balloon inflation is performed 3 times for 1 minute each at 6 atm (nominal pressure). The balloon is deflated for 1 minute between inflations. After this initial injury, the same balloon is used to perform the injury on the contralateral external iliac artery in a similar fashion.
◇ Infection step :
   1. Attach a 3-way stopcock to each end of the flexible extension tubing.
   2. Fill the Leveen Inflator with a 50/50 mixture of saline and contrast agent. Purge the inflation device of air. Attach the inflation device to the flexible extension tubing, via one of the stopcocks.
   3. Attach the stopcock at the free end of the flexible extension tubing to the INF port on the catheter.
   4. Set the proximal stopcock to allow filling of the flexible extension tubing with the desired volume of drug solution.
   5. With the distal stopcock open to air, and the proximal stopcock set to allow connection between in infusion device and the extension tubing, complete the filling operation within the flexible extension tubing using solution from the infusion device.
   6. Close the distal stopcock to air, thereby allowing connection between the extension tubing and the catheter.
   7. Prior to inserting the guide wire into the e-Med Microfuse, Annular or Channel balloon Catheters, flush the wire lumen of the catheter with sterile saline using the 20 ml syringe.
   8. The balloon is introduced via the carotid into the external iliac artery immediately distal to the origin of the internal iliac artery under radioscopic guidance.
   9. Air (equivalent to the dead space, approximately 900µl) and the viral solution (500µl) is chased using a Leveen Inflator with 50/50 mixture of saline and contrast agent.
   10.The pressure will be monitored and kept at 3 atm.
   11.The balloon is deflated when contrast dye in the balloon will appear. Therefore, infusion time can fluctuate from one rabbit to another.
   12.The balloon is introduced in the contralateral iliac artery.
   13.New extension tube is filled with viral solution and procedure is repeated.
   14.After this second transfer the balloon is deflated and removed.
   15.The carotid is ligated with a 3.0 silk (Ethicon).
   16.The wound is closed with one subcutaneous suture (silk 4.0) and a cutaneous suture (suturamid 2.0).The animals are allowed to recover from anesthesia and replaced in their cage.

All animals dead during the experiment are notified.

### 3.1.3 EXAMINATIONS PERFORMED

Three days after transfer animals are killed by pentobarbital overdose. The treated arterial segments are harvested for histochemical analyses and dipped in 3.7% Formalin solution. At this step, samples can be transferred out of the L₂ laboratory to the anatomohistology unit.

### 3.2. Material and methods for gene transfer in atheromatous rabbits

### 3.2.1 ANIMALS

- Species : Rabbits.
- Strain : New Zealand White.
- Sex : Male.
- Characterization :Conventional.
- Body weight range at initiation of treatment : 4,0 Kg.
- Experiments were reviewed and approved by internal ethical committee (Approval n°95-15).

### 3.2.2 TREATMENT

| **Adenovirus description** | | | |
|---|---|---|---|
| Construct | Batch | Particle number | Titer |
| AV_{1.0}CMV β-Gal | V120 | 4.1.10¹² PV/ml | 4.5.10¹⁰ pfu/ml |

- Buffer: PBS containing 10% glycerol
- Storage :- 80°C

### Adenovirus preparation

• Prepare PBS solution as followed:

| | |
|---|---|
| PBS pH7,4 | 45 ml |
| Glycerol 10% | 5 ml |
| MgCl₂ 0,5M 0,1% | 50 µl |

• Thaw rapidly the adenoviral aliquots at room temperature and dilute them in the PBS solution described above.
• Dilutions are prepared just before use in order to avoid any virus degradation with prolonged storage.

### Animal groups and treatment

Adenovirus is administered as described in the following table :

| Group | n | Injected dose (PV/artery) | Infusion time | Pure adeno volume (µl) | PBS volume (µl) |
|---|---|---|---|---|---|
| 1 | 6 | 1.10¹² | 2min | 244 | 256 |
| 2 | 4 | 0 | 2min | 0 | 500 |

◇ At day 1 :
   Rabbits are weighted and blood samples are taken.
   Animals are fed 140g daily with a 1.33% cholesterol diet provided par U.A.R (Usine d'Alimentation Rationelle. 7,rue Galliéni. Villemoison. 91360 Epinay sur Orge).
◇ At day 8 :
   Blood samples are taken.
◇ At day 15 : Injury step :
   - Blood samples are taken.
   - Following placement in the restraining cage and after disinfection, intramuscular injection of a mixture of Ketamine (50mg/kg) / Xylazine (5 mg/kg) is administered. A 22 gauge catheter is placed in the marginal ear vein and is flushed with sodium heparinate (120 Ul/kg). Blood samples are taken.
   - A 5 Fr. introducer sheath (Vygon) is positioned in the carotid artery. All catheters are subsequently introduced through this sheath.
   - A 3 cm long 2.5 mm angioplasty balloon (Nycomed) is then advanced over a 0.014" guide wire (Boston Scientific). Balloon inflation is performed 3 times for 1 minute each at 6 atm (nominal pressure). The balloon is deflated for 1 minute between inflations. After this initial injury, the same balloon is used to perform the injury on the contralateral external iliac artery in a similar fashion.
◇ At day 29 :
   - Blood samples are taken
◇ At day 43 (4 weeks after the initial abrasion): Infection step
   - Blood samples are taken.
   - Following placement in the restraining cage and after disinfection, intramuscular injection of a mixture of Ketamine (50mg/kg) / Xylazine (5 mg/kg) is administered. A 22 gauge catheter is placed in the marginal ear vein and is flushed with sodium heparinate (120 Ul/kg). Blood samples are taken.
   - A 5 Fr. introducer sheath (Vygon) is positioned in the carotid artery. All catheters are subsequently introduced through this sheath.
   - A 2 cm long 2.5 mm channel balloon catheter (Boston Scientific) or e-Med catheter is used for the transfer. 350µl of the viral solution is infused in the catheter (equivalent to the predetermined dead space).
   - The catheter is then advanced over a 0.014" guide wire and inflated with contrast medium at 6 atm 3 times for 1 minute each to perform the second injury. The catheter is then inflated at the same pressure and the viral solution is chased using a syringe pump with 550µl of contrast solution.
   - The infusion time is 2 min. The pressure is monitored and recorded with a pressure transducer.
   - The balloon is deflated, placed in the contralateral artery and the procedure is repeated.
   - The wound is closed with one subcutaneous suture (silk 4.0) and a cutaneous suture (suturamid 2.0).The animals are allowed to recover from anesthesia and replaced in their cage.
◇ At day 46 :
   - All animals are sacrificed.

All animals dead during the experiment are notified.

### 3.2.3 EXAMINATIONS PERFORMED

### Post mortem procedure

Three days after transfer animals are killed by pentobarbital overdose. The treated arterial segments are harvested for histochemical analyses and dipped in 3.7% Formalin solution. At this step, samples can be transferred out of the L2 laboratory to the anatomohistology unit.

### 3.3 HISTOLOGICAL ANALYSIS

After X-Gal incubation arterial segments were maintained in a 3.7% formalin solution until paraffin embedding. Sections (5 µm) for histochemical analysis were routinely processed with Nuclear Red as counterstaining. Macroscopically β-galactosidase expression (" blue arteries ") and microscopic level β-galactosidase expression was also studied in the arterial wall.

### 4. Results

### Summary of the studies in normal rabbits

More than 33% of iliac arteries (6/18 arteries) were found having more than 120 β galactosidase positive cells in 4 sections using the e-Med catheter whereas only 22 (2/9 arteries) and 12% (1/8 arteries) of arteries with the Annular balloon and Channel balloon catheters, respectively. 33% of iliac arteries (6/18 arteries) exhibited between 30 and 120 β-galactosidase positive cells in 4 sections using the e-Med catheter whereas only 22 (2/9 arteries) and 12% (1/8 arteries) of arteries with the Annular balloon and Channel balloon catheters. Only 33% of iliac arteries (6/18 arteries) displayed less than 30 β galactosidase positive cells in 4 sections using the e-Med catheter whereas there was 55 (5/9 arteries) and 75% (6/8 arteries) of arteries with the Annular balloon and Channel balloon catheters, respectively. These data indicated a superior gene transfer in the arterial wall using the e-Med microporous catheter.

### Summary of the studies in atheromatous rabbits

In this more relevant model, more than 71% of iliac arteries (10/14 arteries) displayed more than 120 β-galactosidase positive cells in 4 sections using the e-Med catheter whereas no staining was observed (0/8 arteries) in arteries transduced with the use of the Channel balloon catheters. 14% of iliac arteries (2/14 arteries) showed between 30 and 120 β-galactosidase positive cells in 4 sections using the e-Med catheter (50% for the Channel balloon catheter). Only 14% of iliac arteries (2/14 arteries) exhibited less than 30 β-galactosidase positive cells in 4 sections using the e-Med catheter whereas this applied to 50% (3/6 arteries) of arteries with the use of the Channel balloon catheter. Again, these data indicated a superior gene transfer in the arterial wall using the e-Med microporous catheter.

In addition, gene transfer was homogenous among the transfer length (balloon length is 20 mm). The length mean gene transfers were 14.6 ± 1.1 mm (n=14) and < 4 mm (n=8) using e-Med versus Channel balloon catheters (P<0.05; ANOVA), respectively.

Analysis demonstrates that gene transfer was enhanced in atheromatous versus normal iliac artery. All cells of arterial wall, i.e. endothelial, neointimal and smooth muscle cells were transduced by the adenovirus. There was no cellular inflammatory response due to the catheter-mediated gene transfer.

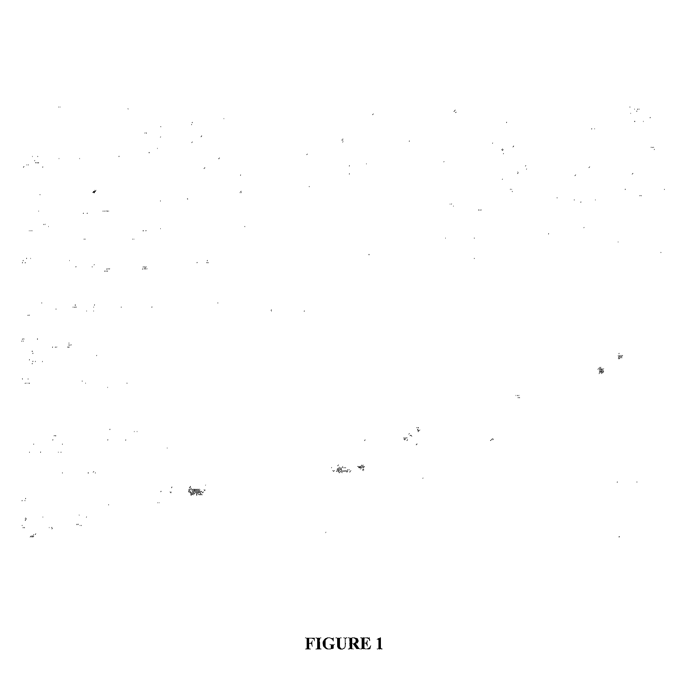

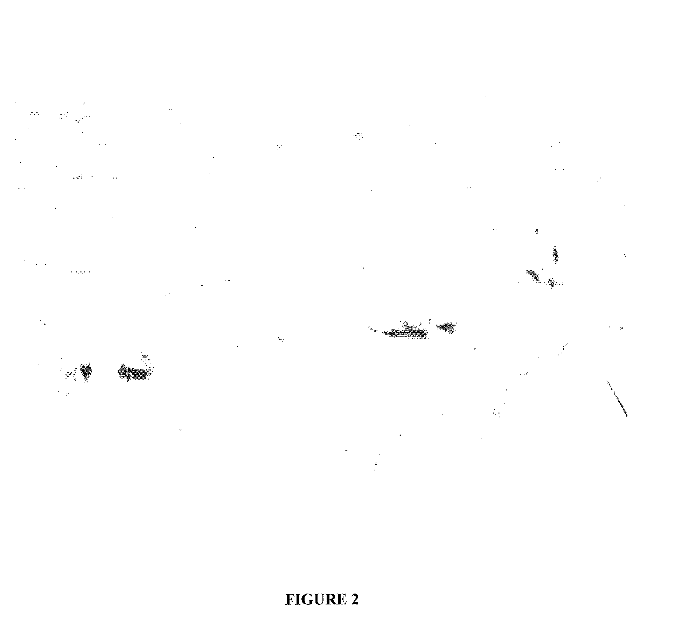

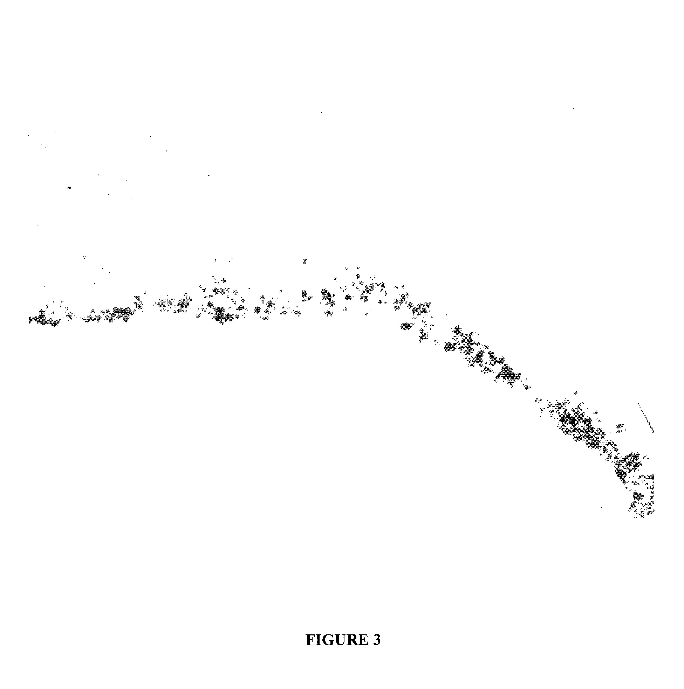

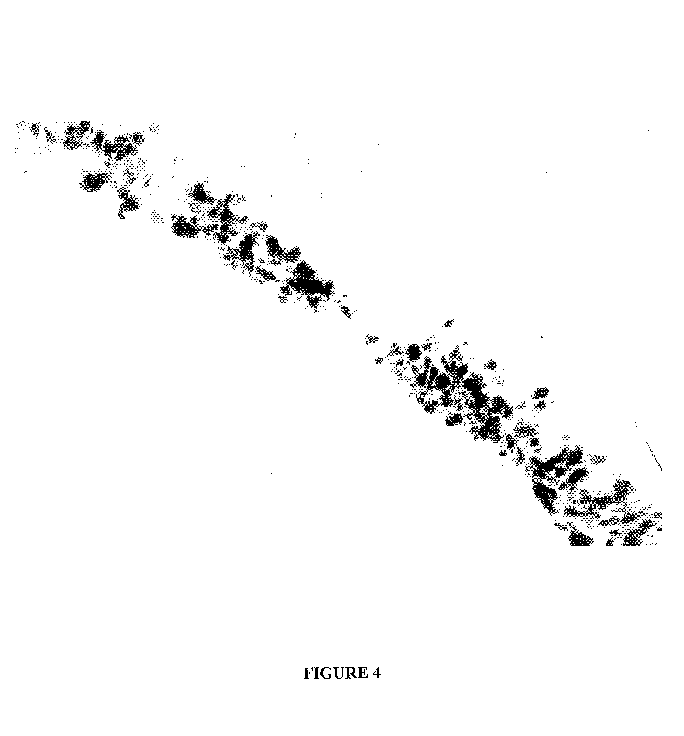

Representative pictures of arterial gene transfer with a Channel balloon and e-Med catheters are shown in figures 1 to 4.

### 5. Discussion

In this work, adenoviral gene delivery to the vessel wall was systematically superior, in term of homogeneity and efficiency, with the use of percutaneous e-Med microporous catheter than that obtained with the Annular Balloon or the Channel balloon, in clinically relevant conditions such as the short delivery time of 2 minutes. These data were observed in both normal and atheromatous rabbit iliac artery model of stenosis. We demonstrated that viral particles penetrate arterial tissue and that transduction of endothelial, neointimal and smooth muscle cells was achieved with high efficiency. We believe that the e-Med microporous balloon will be suitable to deliver therapeutic adenoviral solutions.

### Example 2: Adenoviral-mediated β-Galactosidase Gene Transfer in a Porcine Model of Coronary Artery Restenosis

### Summary

This report summarizes the methods and results from a study which evaluated the ability of 4 local delivery devices to deliver a marker gene, β-Galactosidase (β-Gal), encoded into an adenoviral vector (AV 1.0 CMV β-Gal / 10¹² virus particles (vp)/artery), immediately prior to stent placement in the atherosclerotic left anterior descending (LAD) and left circumflex (LCX) coronary arteries of hypercholesterolemic pigs. Reported here also are the results of an investigation into the possible influence of arterial branches on the transfer of the marker gene into the arterial wall.

The devices tested were: 1. Nycomed "Annular Balloon" catheter (Nycomed Medical Systems, Paris, France), 2. e-Med "Microfuse" catheter (e-Med, St. Paul, MN), 3. IVT "Infiltrator" (Interventional Technologies, San Diego, CA) and 4. Boston Scientific "Channeled Balloon" catheter (Boston Scientific, Natick, MA). The relative efficacy of gene transfer was determined initially by semi-quantitative analysis of the intensity of apparent staining of arteries with β-Gal following incubation in X-Gal solution for 18 hr. Quantitative measurement of the area of staining was also performed. Microscopic quantification of cells staining for β-Gal was used to define the absolute efficacy of gene transfer (Gencell histology group, Vitry). In general, while a relatively high degree of apparent staining was observed in both the LAD and LCX across all treatment groups, quantification of cells staining for β-galactosidase suggested that overall, only low transfer of the marker gene was achieved, and that the e-Med "Microfuse" catheter more effectively transferred the gene than the other devices tested. Statistical analysis of the number and distribution of arterial branches along the LAD and LCX arterial segments exposed to the marker gene detected significantly more branches in the LAD segments than in the LCX segments. Analysis of variance, however, found no difference in the number of branches per arterial segment when they were grouped by local delivery device.

### Materials and methods

### Animal studies

This study utilized a porcine model of restenosis, incorporating plaque creation and stent deployment in the LAD and LCX coronary arteries of hypercholesterolemic swine. Plaque creation was caused by a combination of atherogenic diet and acute balloon over-stretch and denudation of the artery. This process achieves about a 50% arterial stenosis within 4-5 weeks. Four to nine weeks post plaque creation (after acute artery over-stretch), a vascular stent, sized to approximate the artery area, was expanded at the site of pre-established lesion. This procedure has been shown to result in neointima formation and restenosis of the artery.

All *in vivo* procedures used in these studies were performed in accordance with the Animal Welfare Act Regulations and with the Guide for the Care and Use of Laboratory Animals.

Thirty adult male, Yucatan minipigs (31.8 ± 1.5, range: 21.1 - 57.0 kg, Sinclair Research Center, Columbia, MO) were used for this study. Twenty-eight pigs were randomly assigned to one of four treatment groups which differed only by the choice of local delivery device, and received the adenovirus encoded with the β-Gal gene. Two pigs received only buffer, and served as controls.
Three to five days prior to the initial procedure, and continuing throughout the study, pigs received an atherogenic diet (20% saturated fat, 2% cholesterol, Dyets, Bethlehem, PA) and aspirin (Ascriptin, 250 mg, bid). At the time of balloon over-stretch, the animals received an initial 3000 IU of heparin as an iv bolus followed by 1000 IU iv every 20 minutes. A 0.95 mg/min lidocaine infusion was started and maintained for the duration of the procedure. Nitroglycerin was administered at 287 µg/min until a 5 mm decrease in blood pressure was observed; it was then decreased to 116 µg/min and maintained throughout the experiment.
The arterial vasculature was accessed by a right lateral neck incision and exposure of the right carotid artery. A blood sample for determination of arterial blood gases, pH and ion concentration (I-stat, Sensor Devices, Inc., Waukesha, WI) and cholesterol level (Cholestrak, ChemTrak, Sunnyvale, CA) was obtained. With fluoroscopic guidance, an 8F Guide catheter (FCR3.5, SCIMED ) was advanced to the coronary ostia and positioned so that the LAD could be engaged. A 0.014 in. PTCA (Percutaneous Transluminal Coronary Angioplasty) guide wire was then advanced into the artery and an intravascular ultrasound (IVUS) catheter (Ultracross, 30 MHz, SCIMED) was advanced to the end of the wire. IVUS studies were performed utilizing an automatic withdrawal rate of 0.5 mm/sec. Imaging runs were performed over a distance of 5 cm. The IVUS images were continuously recorded and stored on ½-in high-resolution VHS tapes. After recording the imaging run, the IVUS transducer was again advanced to the start point and an angiogram of this position was recorded. Angiograms were subsequently recorded every 1 cm over the 5 cm imaging distance for the purpose of accurately positioning the PTCA balloon. Arterial lumen measurements were made every 5 mm for 2 cm along a segment of artery which was relatively devoid of branches. The average lumen area along this segment was determined, and a balloon diameter was selected that could achieve a calculated 1.8x over-stretch of this area. Using the angiograms as a guide, a non-compliant PTCA balloon (NC Express Plus, SCIMED ) was advanced to the desired segment of artery and was expanded to the calculated diameter for 20 sec. The balloon was then deflated and the artery was allowed to rest for 1 min. This cycle was repeated twice and was followed by 3 gentle rubs of the endothelium with the balloon inflated to a diameter that produced slightly less than a 1.8x over-stretch of the artery. The balloon and guide wire were withdrawn and the guide catheter was repositioned so that the LCX could be engaged, and the entire procedure was repeated.
The carotid artery was ligated and the neck incision was closed in 2 layers, using 3-0 vicryl for each layer. Animals were placed in an intensive care unit until recovered from anesthesia and were then returned to their home cages.
Three days prior to the gene delivery procedure, pigs received Ticlopidine (250 mg, bid), which was continued for a total of 6 days. 39 ± 1 (range: 29 - 62) days after the initial arterial injury, pigs were brought back into the catheterization lab and under the conditions described above, the created stenosis in the LAD and the LCX coronary artery was located. An IVUS run was performed as described above. The artery and lumen cross sectional area (CSA) along a 2 cm length of stenotic artery were measured, from which the artery and lumen diameters were calculated. This allowed selection of the delivery device size and a balloon/stent catheter for stent deployment. Lumen area measurements determined delivery device size selection; artery area measurements determined balloon/stent size selection. Under sterile conditions, the local delivery catheter was filled with the marker gene and contrast solution as described below, and the device was used in accordance with each manufacturer's instructions. A new delivery catheter was used for each artery. After delivery of the marker gene, a balloon expandable NIR Stent (NIR PRIMO, SCIMED) pre-mounted on a non-compliant balloon was advanced to the site of gene delivery and expanded to approximate the artery area. An angiogram was obtained to confirm successful stent deployment. The animals were again placed in an intensive care unit until recovered from anesthesia and were then returned to their home cages.
Three days following gene delivery and stent deployment, animals were euthanatized with an overdose of pentobarbital and the heart was removed. The LAD, LCX and right coronary arteries were dissected free from the underlying myocardium, and prepared for histological analysis as described below.

### Preparation of the local delivery catheters for marker gene delivery

Preparation of the adenovirus for local delivery was performed under a fume hood. The stock adenovirus solution (AV_{1.0} CMVβGal/V143) was supplied in aliquots of 5 x 10¹² virus particles (vp)/ml. A working solution (10¹² vp) was prepared by diluting 200 µl of stock solution with 300 µl of PBS buffer solution (pH 7.4 containing 10% (v/v) glycerol and 0.1% (v/v) 0.5M MgCl₂). The adenovirus vector containing the β-Gal marker gene was sandwiched between 2 columns of 50% contrast media solution. Under sterile conditions, a 30 inch extension set (Abbott Laboratories, North Chicago, IL), fitted at one end with a 3-way stopcock, was filled with 50% contrast media solution. A 6 inch extension set (Maxxim Medical, Athens, TX), fitted at each end with a 3-way stopcock, was filled with the adenoviral vector as follows. With the distal stopcock positioned so that the tubing was opened to air, 500 µl of the adenoviral solution was carefully loaded into the tubing until a small air bubble was observed in the hub of the proximal stopcock. The distal stopcock was then positioned so that the tubing was closed to air, and the long extension set containing the contrast media was attached to the proximal stopcock, ensuring that all the air was expelled from the stopcock. The dead-space volume of a local delivery catheter was then determined by slowly filling the catheter with 50% contrast media. The volume of catheter dead space plus the 500 µl of adenovirus solution comprised the total volume of solution infused into the artery. The adenovirus end of the extension set combination was then secured to the local delivery catheter, and the unit was then taken to the catheterization suite for administration to the animal. Infusion times and/or balloon inflation pressures were set according to the manufacturer's instructions for each delivery device.

### Preparation of arteries for histology

Three days following gene transfer the animals were euthanatized with an overdose of sodium pentobarbital, and the heart was removed. The LAD, LCX and right coronary arteries were carefully removed and placed in cold (4°C) 3.7-4.0% formaldehyde in Dulbecco's PBS (DPBS) for 3 hours. The stents were carefully removed and the arteries were placed in a 20% sucrose solution in DPBS. The tissues were stored in this solution at 4°C for up to 30 hr prior to being stained for β-gal.

In preparation for staining, the arteries were subjected to a series of washes in 2mM MgCl₂/DPBS (see appendix for details) and were then placed in a 2mM MgCl₂/DPBS permeabilization solution containing 0.01% (final conc.) deoxycholic acid and 0.02% (final conc.) Nonidet P40 for 30 min at 4 °C. The tissues were stained in X-gal solution (see appendix) for 18 hr at 37 °C with constant agitation. β-galactosidase reacts with the X-gal to form an insoluble blue precipitate, thus staining blue, those tissues which express the enzyme.

Following incubation in the staining solution, the arteries were rinsed twice with DPBS, and photographed for semi-quantification of β-Gal staining. All arteries were photographed after staining using EZ-Trac, Inc. software on a Dynex 386-25 PC with a Javelin MDS Solid State CCD camera. Images were captured each week from the tissues that were collected and stained that week. A metric ruler was placed in each image field for area calibration when the area of vascular staining was determined. After image capture, tissues were returned to 3.7-4.0% formaldehyde in DPBS and stored at 4 °C until microscopic determination of β-gal expression could be performed in paraffin sections.

Histological sections (5µ) were routinely counter stained with Nuclear Red. Eight sections were cut from each stented segment of the artery, and 4 views from each section were analyzed. Microscopic determination of LacZ β-gal expression was assessed in the arterial wall and in the perivascular tissues (small vessels, fibroblastic cells, macrophages and myocardial muscle cells).

### Analysis of delivery device balloon diameter to lumen diameter ratios, the number of arterial branches and relative distribution of branches in treated segments of LAD and LCX coronary arteries.

The delivery device balloon diameter to lumen diameter ratio for each treated artery was determined. Ratios were calculated by dividing the expanded balloon diameter by the lumen diameter.
To determine if branches along the treated segment of the artery had a role in the uptake of the marker gene by the arterial wall, the number of branches in each treated segment of the LAD and LCX coronary arteries was counted. The branches were counted by viewing that segment of the IVUS tapes corresponding to the position of the delivery device balloon as determined by the length of the balloon.

The relative position of the branches counted in the LAD arteries was subsequently determined. To perform this analysis, the treated segment of the artery was divided into 2 halves, a distal half and a proximal half. Then using the length of the transfer region of the balloon as the length of the treated segment, the position of each branch was normalized relative to the total length of the treated area and expressed as a percent of the treated length. Those branches positioned from 0-50% were placed in the distal half and those positioned from 51-100% were placed in the proximal half. The number of branches falling in each half was then expressed as a percent of the total number of branches counted in that artery.

### Statistics

Data are presented as the mean ± sem. Student's t-test for unpaired data was used to test for differences between groups. Analysis of variance (ANOVA) was used to test for differences within groups. Correlations were performed by linear regression using the least squares method. Results were considered statistically significant at *P<0.05* (two-tailed).

### Results

### Summary of delivery device characteristics.

A summary of some of the more pertinent delivery device characteristics supplied by the manufacturers is presented in Table 1. Table 2 presents data comparing parameters such as balloon inflation pressure, volume of infusate, and the time of infusion, generated during use of the different delivery devices in this study. Table 2 also provides a summary of the microscopic quantification of β-Gal staining in the LAD and LCX coronary arteries.

### Intensity of apparent staining.

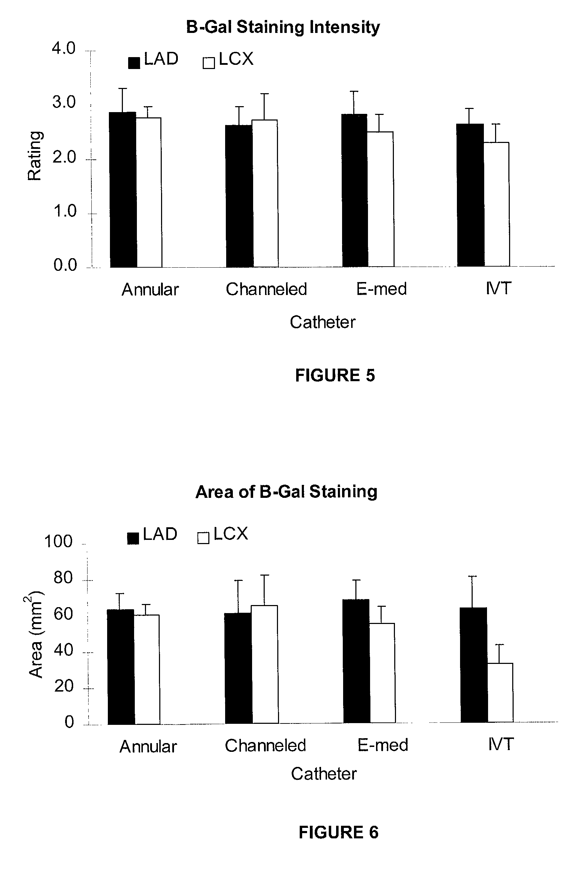

A semi-quantitative rating scale of 0 - 4 was devised to rate the intensity of apparent β-gal staining of the arteries. This is the staining observed in arteries prior to their embedding in paraffin. Photographs of arteries from a given staining run were analyzed together. Arteries demonstrating no apparent staining were rated 0, while those arteries demonstrating the greatest amount of apparent staining were rated 4. The photographs were rated by 3 independent observers who were unaware of the treatment group to which the arteries belonged. The scores were averaged and are presented in FIGURE 5 below. In general, a relatively high degree of apparent staining was observed in both the LAD and LCX across all treatment groups, and no significant difference (p = 0.96) was seen among the different groups. No staining was evident in the right coronary artery from any animal.

### Quantification of the area of apparent β-Gal staining.

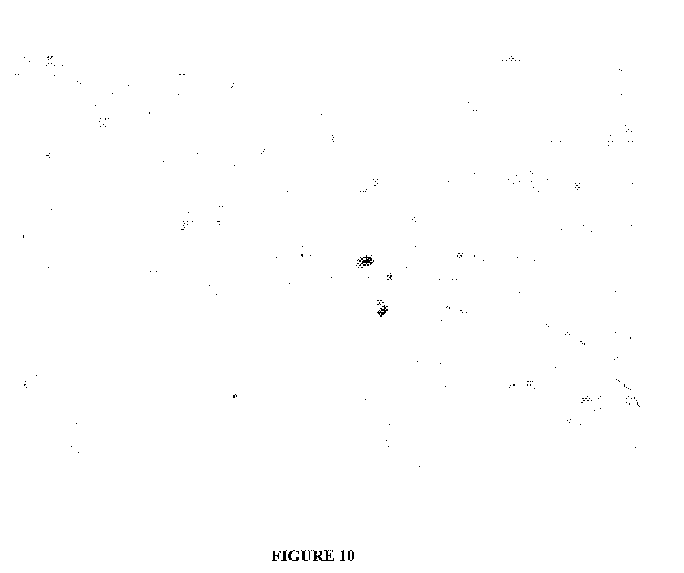

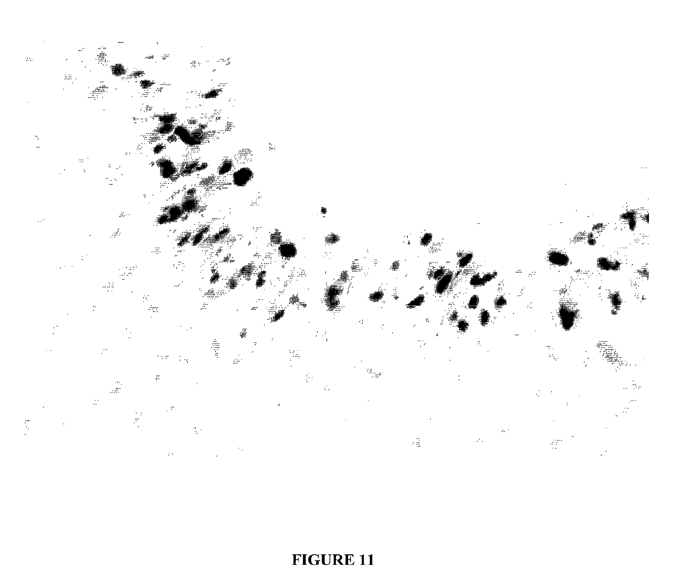

Photographs of the samples were used to measure the area of staining. Using the metric ruler recorded in each image, the system was calibrated to measure in millimeters, and the area of visible blue staining was traced. The area measurements were averaged by artery for each treatment group, and are presented below in FIGURE 6. For the LAD, the area of staining ranged from 61.4 ± 18.0 to 68.5 ± 10.7 mm², while for the LCX, the area of staining ranged from 32.8 ± 10.4 to 65.7 ± 16.9 mm². There was no statistically significant difference (p = 0.67) in the area of artery stained between the LAD or LCX, among any of the devices tested.
Representative pictures of arterial gene transfer with a Channel balloon and an e-Med catheters are shown in figures 10 and 11.

### Microscopic quantification of β-galactosidase expression.

Recombinant adenovirus, containing a nucleus-localized variant of β-galactosidase (LacZβ-gal) was used to quantitate gene transfer. In the arterial wall, LacZ β-gal expression was detected in endothelial cells, neointimal cells and necrotic cells of undetermined character. LacZ β-gal expression also was observed in the perivascular tissues where it was localized to macrophages, fibroblastic cells and myocardial cells.
The e-Med catheter transfected statistically significantly more arteries than the other catheters tested (Table 5). More than 70% of the coronary arteries were transduced using the e-Med catheter whereas 38%, 17% and 20% of arteries were transduced with the Annular Balloon, Channeled Balloon and the Infiltrater catheters, respectively. In addition, the average number of cells staining positive for β-gal in the transfected arterial segments was significantly greater for the e-Med catheter than for the other catheters. The data indicate a superior gene transfer in the arterial wall using the e-Med Microfuse catheter.

### Cholesterol levels.

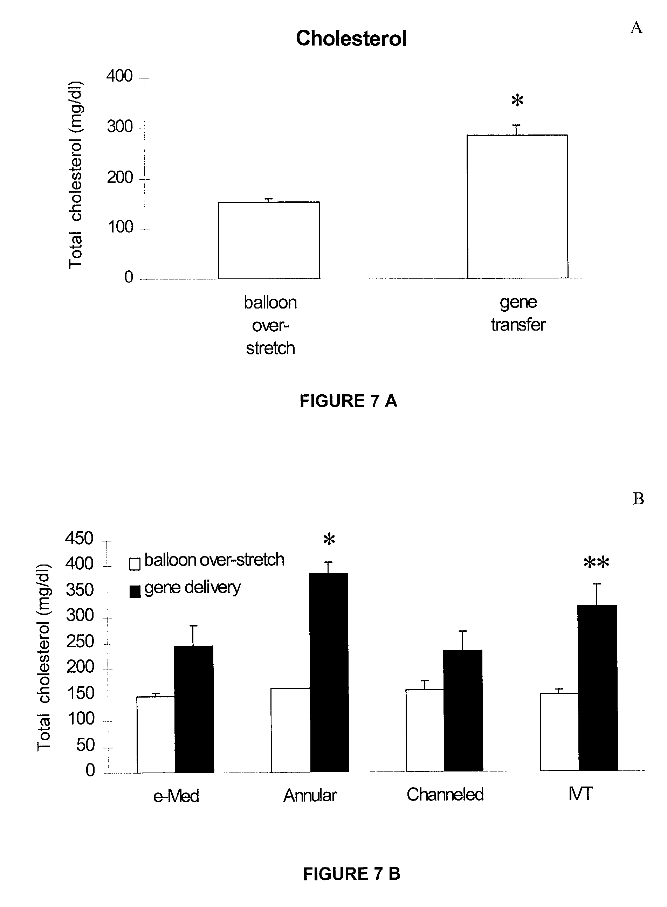

The mean total cholesterol level of the pigs at the time of balloon over-stretch was 153.8 ± 5.2 mg/dl. At the time of gene delivery and stent deployment, the mean total cholesterol had increased significantly (p < 0.001) to 284.8 ± 22.1 mg/dl (FIGURE 7A). Analysis of variance showed that the total cholesterol levels at the time of plaque creation did not differ significantly among the treatment groups. However, at the time of gene transfer and stent expansion, significant differences were observed. In the pigs treated with the Annular balloon, cholesterol was significantly higher (p = 0.03) than in the pigs treated with the Microfuse or Channeled balloons. Likewise, the average cholesterol level in the pigs treated with Infiltrator balloon was significantly greater (p = 0.03) than that in the pigs treated with the Channeled balloon (FIGURE 7B). When the relative changes in cholesterol, from the time of plaque creation to gene transfer, were analyzed, a significant difference (p = 0.03) was observed between only the Annular group (245.5 point increase) and the Channeled balloon group (47.7 point increase). Since the arteries from either of these groups demonstrated very little staining, it is unlikely that differences in cholesterol can account for the differences in arterial staining observed between the e-Med group on the one hand, and the other groups.

### Summary of balloon diameter to lumen diameter ratios.

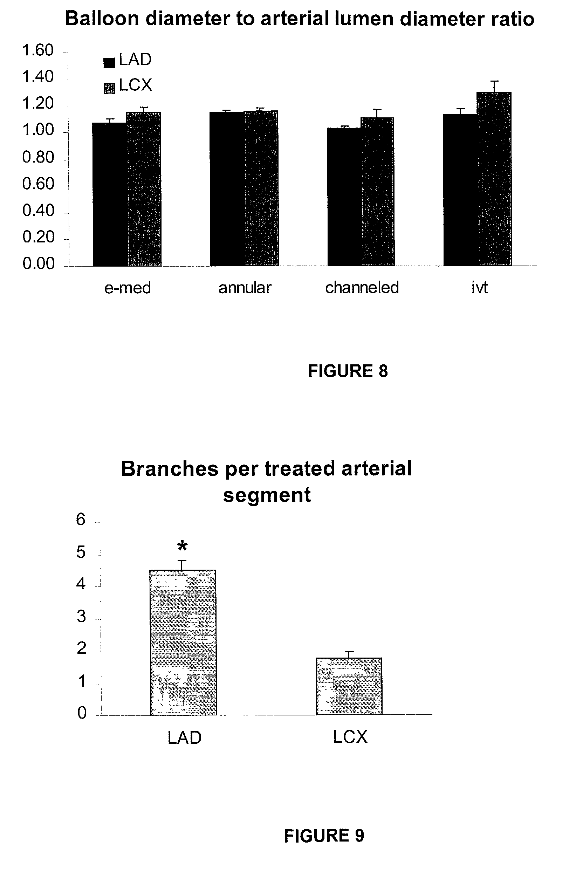

Since the possibility existed that a difference in the balloon diameter to lumen diameter ratios or, alternatively, the amount of dilation experienced by the artery during gene delivery, might affect the uptake of the marker gene by the arterial wall, an analysis of the balloon diameter to lumen diameter was performed. The arteries were grouped according to the local delivery device with which they were treated. No significant difference (p = 0.09) in the balloon diameter to lumen diameter ratio among the 4 groups was found (FIGURE 8). The balloon to lumen diameter ratios were then subjected to linear regression analysis relative to arteries staining positive for β-Gal. No correlation was found between arteries staining positive for β-Gal and the balloon to lumen diameter ratios. The data are presented in table 3.

### Summary of arterial branch analysis.

To ensure that the number of branches or their distribution was not different for any of the arteries subjected to gene transfer, an analysis of branch number and their relative distribution in each treated arterial segment was undertaken. The branches were counted by viewing that segment of the IVUS tapes corresponding to the position of the delivery device balloon as determined by the transfer region of the balloon. A significant difference (p < 0.001) was found between the number of branches found in the LAD and those counted in the LCX (FIGURE 9). Table 4 shows the data from an analysis of the relative position of the branches counted in the LAD arteries. No significant difference was observed in either the number of branches, normalized for the length of transfer region (p = 0.1, data not shown), or the relative position of branches (p = 1.0) among any of the treatment groups.

### Discussion.

The two most significant findings of this study are: 1) that while a relatively large amount of apparent staining was seen in both the LAD and LCX arteries from all the delivery device groups, overall, very little cellular staining was observed microscopically. 2) As measured by the total number of arteries with cells staining positive for β-Galactosidase, the e-Med Microfuse catheter more effectively transferred the gene than did the other 3 devices.

In addition only the e-Med catheters allowed complete cellular transduction along the arterial segment corresponding to the length of the balloon.
Since the total cholesterol level can influence the composition of an atherosclerotic lesion in pigs (Recchia, et al., . Aterioscler Thromb Vasc Biol. 1995; 15:924-929), it is possible that differences in plaque composition might affect the uptake of a gene by the artery. In this study, it seems unlikely that lesion composition, as projected from cholesterol levels, could explain the observed differences in artery staining. At the time of plaque creation (acute balloon over-stretch), no significant difference in the cholesterol level among the 4 groups was evident. At the time of gene transfer, the mean total cholesterol of the pigs in the Annular group was 407 mg/dl which was significantly higher than that of the pigs in either the e-Med group or the Channeled group (FIGURE 7B). However, the total number of arteries staining positive for β-Gal was markedly higher in the e-Med group compared to both the Annular and Channeled groups. While the cholesterol level was significantly higher in the Annular group than in the Channeled group, the number of arteries staining positive for β-Gal did not differ between these two groups (Table 2).

The degree of arterial injury is directly linked to relative amount of acute balloon over-stretch imposed upon a naive artery (Bonan et al., Am Heart J. 1993; 126:1330-1340). Although the extent to which this is valid in a stenosed artery is debated (Carter, et al., . J Am Coll Cardiol. 1996; 27:1270-1277.), re-injury of the artery during gene transfer could alter arterial uptake the gene. During gene transfer it is therefore important to select a delivery device balloon size that approximates the lumen diameter to minimize arterial wall injury. An analysis of the delivery device balloon diameter to arterial lumen diameter ratios showed no significant differences among any of the groups. Therefore, it seems unlikely that differences in balloon to lumen ratios affected transfer of the gene.

A preliminary report showed a relatively large amount of viral leakage, as evidenced by staining of the myocardium close to the LAD and LCX, and suggested that the numerous collaterals observed microscopically could account for this observation. We undertook an investigation of the number of branches in the arterial sections exposed to the gene. Significantly more branches were seen in the LAD arteries than in the LCX arteries. But when analyzed by local delivery device group, no significant differences were noted among the groups. It is possible that the number of branches is responsible for the observed myocardial staining and may have contributed to the overall low level of vascular staining, but it is unlikely that these branches can account for the differences seen in vascular staining.

In summary, the 4 local delivery devices (Nycomed "Annular Balloon" catheter, e-Med "Microfuse" catheter, IVT "Infiltrator" and Boston Scientific "Channeled Balloon" catheter), all resulted in a relatively large degree of apparent vascular staining, but only a low amount of microscopically quantifiable cellular staining following local delivery of an adenoviral-mediated marker gene, β-Gal, to the arterial wall of injured, atherosclerotic LAD and LCX coronary arteries of hypercholesterolemic swine. Of the 4 devices, the e-Med Microfuse catheter resulted in the greatest number of arteries staining positive for β-Gal.

These results show clearly that only the combination of the e-Med Microfuse catheter and of adenovirus comprising a gene achieves an efficient transfection of the arterial cells. Naked DNA would not achieve a comparable level of transduction.

**Table 1.**

| *Delivery Device Characteristics* | | | | | | |
|---|---|---|---|---|---|---|
| Delivery Device | Type | Flow thru | Working length (cm) | Transfer region (cm) | pore size | # of pores |
| e-Med Microfuse | intra-luminal | no | 2.0 | 1.7 | <1µ | >10⁶ |
| | | | | | | |
| Boston Scientific Channeled | intra-luminal | no | 2.0 | <2.0 | 100µ | 9 |
| | | | | | | |
| Nycomed Annular | intra-luminal | yes | 2.0 | 1.0 | annular chamber | 0 |
| | | | | | | |
| IVT Infiltrator | intra-mural | no | 1.5 | <1.5 | 100µ | 21 |

**Table 2.**

| *Artery staining and device characteristics for gene delivery.* | | | | | | | | | | |
|---|---|---|---|---|---|---|---|---|---|---|
| Delivery device | + staining for β-Gal | | Inflation pressure (atm) | | volume infused (ml) | | infusion rate (ml/hr) | | length of infusion (sec) | |
| | LAD | LCX | LAD | LCX | LAD | LCX | LAD | LCX | LAD | LCX |
| e-Med Microfuse | 7 | 5 | 3.5 ± 0.5 | 3.5 ± 0.5 | 1.3 ± 0 | 1.8 ± 0.4 | 108 ± 13 | 152 ± 22 | 45 ± 5 | 44 ± 6 |
| | | | | | | | | | | |
| Boston Scientific Channeled | 3 | 0 | 4.5 ± 0.7 | 4.5 ± 0.7 | 0.8 ± 0 | 0.7 ± 0 | 27 ± 3 | 30 ± 2 | 90 ± 0 | 90 ± 0 |
| | | | | | | | | | | |
| Nycomed Annular | 2+ | 0 | 10.0 ± 0 | 10.0 ± 0 | 0.8 ± 0 | 0.8 ± 0 | 12 ± 0 | 12 ± 0 | 250 ± 3 | 248 ± 2 |
| | | | | | | | | | | |
| IVT Infiltrator | 1 | 2 | 2.0 ± 0 | 2.0 ± 0 | 1.0 ± 0 | 1.0 ± 0 | 122 ± 1 | 118 ± 2 | 30 ± 0 | 30 ± 0 |

**Table 3.**

| *Artery staining and balloon diameter to lumen diameter ratios.* | | | | |
|---|---|---|---|---|
| Delivery device | Number of arteries staining + for β-Gal | | Balloon diameter to lumen diameter ratio | |
| | LAD | LCX | LAD | LCX |
| e-Med Microfuse | 7 | 5 | 1.08 ± 0.03 | 1.15 ± 0.04 |
| | | | | |
| Boston Scientific Channeled | 3 | 0 | 1.04 ± 0.02 | 1.11 ± 0.07 |
| | | | | |
| Nycomed Annular | 2+ | 0 | 1.15 ± 0.02 | 1.16 ± 0.03 |
| | | | | |
| IVT Infiltrator | 1 | 2 | 1.14 ± 0.05 | 1.31 ± 0.09 |

**Table 4.**

| *Summary of the number of branches and their relative distribution in LAD arteries: Analysis by local delivery device.* | | | | | |
|---|---|---|---|---|---|
| Delivery device | Total number of branches | Distal branches | Percent of total | Proximal branches | Percent of total |
| e-Med Microfuse | 3.2 ± 0.5 | 1.9 ± 0.2 | 60 ± 7 | 1.4 ± 0.4 | 40 ± 7 |
| | | | | | |
| Boston Scientific Channeled | 4.4 ± 0.5 | 1.9 ± 0.3 | 43 ± 5 | 2.6 ± 0.4 | 57 ± 5 |
| | | | | | |
| Nycomed Annular | 2.2 ± 0.5 | 1.0 ± 0.3 | 56 ± 16 | 1.2 ± 0.4 | 44 ± 16 |
| | | | | | |
| IVT Infiltrator | 4.7 ± 0.4 | 2.2 ± 0.7 | 43 ± 13 | 2.5 ± 0.5 | 57 ± 13 |

**Table 5.**

| *Gene transfer efficiency. Number of detected Lac Z positive cells identified in all artery segments after adenoviral delivery with each balloon* | | |
|---|---|---|
| Device | # arteries with Lac Z expression | Arterial gene transfer efficiency (# LacZ positive cells in arterial segments) |
| e-Med (n=17) | 12 | 41 +/- 19* |
| Annular (n=13) | 3 | 3+/- 1 |
| Channel (n=17) | 5 | 2+/- 1 |
| Infiltrator (n=13) | 3 | 1+/- 1 |

| | | |
|---|---|---|
| * Signifies p<0.05 compared to other balloons; Values in parenthesis indicated number of arteries exposed to gene transfer catheter. | | |

## Claims

1. Method for delivering a gene of interest to a vascular tissue comprising the following steps:
- inserting into the said vascular tissue a catheter in fluid communication with an inflatable balloon formed from a microporous membrane, and
- delivering to the said vascular tissue through the catheter a solution containing a vector comprising a gene of interest and capable of expressing said gene into said vascular tissue.

2. Method according to claim 1 wherein the vector is a viral vector.

3. Method according to claim 1 wherein the vector is an adenoviral vector.

4. Method according to claim 1 to 3 wherein the microporous membrane pores sized from about 10 Å to about 1µ and a pore density from about 10⁴ pores/cm² to about 10¹¹ pores/cm².

5. Method according to claim 1 to 4 wherein the balloon is inflated.

6. Method according to claim 1to 5 wherein the vascular tissue is an atherosclerotic artery.

7. Method according to claim 6 wherein the vascular tissue is connected with an ischemic tissue or an ischemic organ.

8. Method according to claim 1 wherein the gene is a suicide gene.

9. Method according to claim 8 wherein the suicide gene is a thymidine kinase gene or a cytosine desaminase gene.

10. Method according to claim 1 wherein the gene is a gene encoding an angiogenic factor.

11. Method according to claim 10 wherein the said angiogenic factor is FGF or VEGF.

12. Method for treating a vascular disease comprising the following steps:
- inserting into an vascular tissue a catheter in fluid communication with an inflatable balloon formed from a microporous membrane, and
- delivering to the said vascular tissue through the catheter a solution containing a vector comprising a gene of interest and capable of expressing said gene into said vascular tissue.

13. Method for treating restenosis comprising the following steps:
- inserting into an atherosclerotic artery a catheter in fluid communication with an inflatable balloon formed from a microporous membrane, and
- delivering to the said artery through the catheter a solution containing a vector comprising a suicide gene and capable of expressing said gene into said atherosclerotic artery.

14. Method according to claim 13 wherein said suicide gene is a thymidine kinase gene or a cytosine desaminase gene.

15. Method for treating an ischemic tissue comprising the following steps:
- inserting into an artery connected with said tissue a catheter in fluid communication with an inflatable balloon formed from a microporous membrane, and
- delivering to the said artery through the catheter a solution containing a vector comprising a gene encoding an angiogenic factor and capable of expressing said gene into the cells of said artery.

16. Method for treating a coronary artery disease comprising the following steps:
- inserting into an coronary artery a catheter in fluid communication with an inflatable balloon formed from a microporous membrane, and
- delivering to the said artery through the catheter a solution containing a vector comprising a gene encoding an angiogenic factor and capable of expressing said gene into the cells of said artery.

17. Method according to claim 16 wherein said angiogenic factor is FGF or VEGF.

18. Use of a vector comprising a gene of interest and capable of expressing said gene into a vascular tissue, for the manufacture of a medicament for administering a gene of interest to a vascular tissue by intra vascular administration through an inflatable balloon formed of a microporous membrane.

19. Use of a vector comprising a gene of interest and capable of expressing said gene into said vascular tissue, for the manufacture of a medicament for use in the treatment of cardio-vascular diseases by intra vascular administration through an inflatable balloon formed of a microporous membrane.

20. Use according to claim 19 wherein said disease is restenosis.

21. Use according to claim 19 wherein said disease is an ischemic heart disease.

22. Use according to claim 19 wherein said disease is a peripheral artery disease.

23. Use according to claim 19 wherein said gene is a suicide gene.

24. Use according to claim 23 wherein said suicide gene is a thymidine kinase gene or a cytosine desaminase gene.

25. Use according to claim 20 wherein said gene is a gene encoding an angiogenic factor.

26. Use according to claim 25 wherein said angiogenic factor is FGF or VEGF.

27. A kit comprising:
- a catheter in fluid communication with an inflatable balloon formed from a microporous membrane, and
- a vector comprising a gene of interest.

28. Apparatus comprising:
- a catheter in fluid communication with an inflatable balloon formed from a microporous membrane, and
- a vector comprising a gene of interest.

29. Apparatus according to claim 28 for use as medicament.

30. Apparatus comprising:
- a catheter in fluid communication with an inflatable balloon formed from a microporous membrane, and
- a vector comprising a gene of interest, as a combination for simultaneous or sequential use.
